# EUROPEAN PATENT APPLICATION

(11) **EP 2 674 166 A1**
(43) Date of publication of application: **18.12.2013**
(21) Application number: 12172195.5
(22) Date of filing: 15.06.2012
(51) Int. Cl.: A61K 38/22, A61K 38/18, A61K 31/045, A61K 31/341, A61K 9/00, A61K 9/70, A61P 19/00, A61P 35/00

(54) **Use of erythropoietin (EPO) for the local treatment of cancer treatment-associated osteo-necrosis of the jaw**

(71) Applicant: Universitätsmedizin der Johannes Gutenberg-Universität Mainz, 55131 Mainz (DE)
(72) Inventor: Ziebart, Thomas, 55129 Mainz (DE)
(74) Representative: Krauss, Jan

(57) **Abstract**

The present invention relates to the use of erythropoietin (EPO) for the local treatment of bisphosphonate-associated osteonecrosis of the jaw (BP-ONJ) in the context of a cancer therapy involving bisphosphonates, and other osteonecrosis, such as radioosteonecrosis.

## Description

The present invention relates to the use of erythropoietin (EPO) for the local treatment of bisphosphonate-associated osteonecrosis of the jaw (BP-ONJ) in the context of a cancer therapy involving bisphosphonates, and other osteonecrosis, such as radioosteonecrosis.

Bisphosphonate-associated osteonecrosis of the jaw, often abbreviated as BP-ONJ, or localized death of bone tissue, of the jaws is a rare potential complication in cancer patients receiving treatments including radiation, chemotherapy, or in patients with tumors or infectious embolic events. In 2003, reports reported about the increased risk of osteonecrosis in patients receiving these therapies concomitant with and an intravenous bisphosphonate (reviewed in Lehrer S; Montazem A; Ramanathan L; et al. (January 2009). "Bisphosphonate-induced osteonecrosis of the jaws, bone markers, and a hypothesized candidate gene". J. Oral Maxillo-fac. Surg. 67 (1): 159-61).

Bisphosphonates are administered in order to protect the bone during metastasis. Matrix metalloproteinase 2 may be a candidate gene for bisphosphonate-associated osteonecrosis of the jaws, since it is the only gene known to be associated with both bone abnormalities and atrial fibrillation, another side effect of bisphosphonates. As a side-effect, bisphosphonates cause BP-ONJ by inhibiting the activities of osteoblasts and osteoclasts. There is presently no known prevention or treatment for bisphosphonate-associated osteonecrosis of the jaw. According to the German and US guidelines, BP-ONJ is symptomatically treated using antibiotics and disinfecting mouthwashes.

For many years, EPO has been used in the systemic therapy of, for example, anemia in the context of chemotherapy.

Shiozawa et al. (in: Shiozawa Y, Jung Y, Ziegler AM, Pedersen EA, Wang J, et al. (2010) Erythropoietin Couples Hematopoiesis with Bone Formation. PLoS ONE 5(5): e10853. doi:10.1371/joumal.pone.0010853) describe that erythropoietin (EPO) activates Jak-Stat signaling pathways in HSCs which leads to the production of BMPs. Critically, EPO also directly activates mesenchymal cells to form osteoblasts in vitro, which in vivo leads to bone formation. Importantly, EPO first activates osteoclastogenesis which is later followed by osteoblastogenesis that is induced by either EPO directly or the expression of BMPs by HSCs to form bone. These data for the first time demonstrate that EPO regulates the formation of bone by both direct and indirect pathways, and further demonstrates the exquisite coupling between hematopoesis and osteopoiesis in the marrow.

Singbrant et al. (in: Singbrant S, Russell MR, Jovic T, Liddicoat B, Izon DJ, Purton LE, Sims NA, Martin TJ, Sankaran VG, Walkley CR. Erythropoietin couples erythropoiesis, B-lymphopoiesis, and bone homeostasis within the bone marrow microenvironment. Blood. 2011 May 26;117(21):5631-42. Epub 2011 Mar 18) describe erythropoietin (EPO) has been used in the treatment of anemia resulting from numerous etiologies, including renal disease and cancer. They present data demonstrating a previously unrecognized in vivo regulatory network coordinating erythropoiesis, B-lymphopoiesis, and skeletal homeostasis. Importantly, these findings may be relevant to the clinical application of EPO.

Jadu et al. (in: Jadu F, Lee L, Pharoah M, Reece D, Wang L. A retrospective study assessing the incidence, risk factors and comorbidities of pamidronate-related necrosis of the jaws in multiple myeloma patients. Ann Oncol. 2007 Dec;18(12):2015-9. Epub 2007 Sep 5) describe bone necrosis of the jaws as a newly recognized complication associated with the use of bisphosphonates. The true incidence of this complication is unknown and the pathophysiology is controversial. The purpose of this study was to determine the incidence of jaw necrosis among a homogeneous population of multiple myeloma patients receiving the bisphosphonate pamidronate, to investigate risk factors and comorbidities that increase the risk and to characterize the radiographic changes on conventional dental radiographs in terms of type and frequency. The following risk factors were found to be statistically significant: longer duration ofpamidronate therapy (P < 0.001), dental extractions (P < 0.001), cyclophosphamide therapy (P < 0.014), prednisone therapy (P < 0.014), erythropoietin therapy (P = 0.006), low hemoglobin levels (P < 0.001), renal dialysis (P < 0.016) and advanced age (P < 0.001). Radiographic changes produced by the necrotic bone were less evident than the clinically exposed bone.

WO 2005/070451 describes pharmaceutical compositions for topical administration, comprising non-glycosylated erythropoietin (EPO), such as bacterially produced recombinant EPO. The compositions are suitable for use in treatment of a human being or an animal suffering from a disease, disorder or surgical lesion that is alleviated or cured by topical administration of erythropoietin, e.g. a disease or disorder selected from a conjunctivitis, a wound, a bedsore, a bum, an inflammation of the skin, mucous membranes, airways or lungs, an eczema or a skin disorder accompanied by necrosis, by dermatitis, by psoriasis or by diabetes mellitus. The pharmaceutical composition may be in the form of an ointment, a cream, a powder, an emulsion, a gel, a glycerogelatine, a paste, a plaster, a sprayable composition or a lotion.

Since there is no available causal therapy, often a surgical depletion of the necrosis and a plastic coverage is the only treatment. Sometimes, the recrudescence even requires a removal of complete segments of the jaw (partial resection of the jaw). Therefore, new approaches for a therapy of osteonecrosis, and in particular of bisphosphonate-associated osteonecrosis of the jaw are sought after. The present invention fulfills these needs.

Thus, a first aspect of the present invention relates to topically applied erythropoietin (EPO) and the use thereof for the prevention and/or treatment of osteonecrosis in a patient.

A systemic use of EPO during the prevention and/or treatment of osteonecrosis, and in particular of BP-ONJ, is not possible, since
- osteonecrosis, and in particular osteonecrosis of the jaw, is a diseases that is limited to the part of the body that is affected, such as the upper or lower jaws;
- most of the patients are tumor patients, a systemic therapy could lead to a progression of the tumor (Magnusson I, Batich C, Collins BR. New attachment formation following controlled tissue regeneration using biodegradable membranes. J Periodontol. 1988 Jan; 59(1):1-6).
- upon systemic application there is a high risk for EPO-associated diseases and side-effects, such as hypertension, and thus an increased risk for infarction and stroke, increased coagulation. polycythemia, etc. (Lippi G, Franchini M, Favaloro EJ. Thrombotic complications of erythropoiesis stimulating agents. Semin Thromb Hemost. 2010 Ju1; 36(5):537-49. Epub 2010 Jul 14).

During experiments in the context with the present invention, it was found that bisphosphonate-necrosis is associated with damaged solid and soft tissues (Walter C, Klein MO, Pabst A, Al-Nawas B, Duschner H, Ziebart T. Influence of bisphosphonates on endothelial cells, fibroblasts, and osteogenic cells. Clin Oral Investig. 2010 Feb; 14(1):35-41; Ziebart T, Pabst A, Klein MO, Kämmerer P, Gauss L, Brüllmann D, Al-Nawas B, Walter C. Bisphosphonates: restrictions for vasculogenesis and angiogenesis: inhibition of cell function of endothelial progenitor cells and mature endothelial cells in vitro. Clin Oral Investig. 2011 Feb;15(1):105-11). Furthermore, it was surprisingly found that EPO significantly increased the viability of osteoblasts and fibroblasts that were subjected to bisphosphonates. Therefore, it is possible, to use EPO for a therapy in the context of bisphosphonate-necrosis, and in particular during the surgical restoration of BP-ONJ.

Preferred is the EPO and the use thereof according to the present invention, wherein said patient is a cancer patient, and preferably a cancer patient undergoing an anti-cancer bisphosphonate therapy. Preferably, said anti-cancer bisphosphonate therapy is provided in an early disease setting, and even extends to cancer prevention. Cancers to be treated can be selected from many solid cancers, and preferably are selected from genitourinary cancers, lung cancer, breast, bone, colon, and prostate cancer, and other solid tumors, as well as metastatic ("secondary") cancers. Bisphosphonates to be used include, for example, nitrogen and non nitrogen containing bisphosphonates, disodium pamidronate, ibandronic acid, sodium clodronate, and zoledronic acid.

In another aspect of the present invention, said osteonecrosis to be treated and/or prevented is selected from BP-ONJ, and BP-ONJ as a side-effect of an anti-cancer bisphosphonate therapy as described herein, and/or radioosteonecrosis in a patient after radiotherapy. The greatest and most dangerous complication in radiotherapy of the head and neck is without doubt osteoradionecrosis (ORN). It occurs most frequently when the dose of radiation is more than 60 Gy or in the case of patients who receive combined radio and chemo-therapy. ORN occurs in 5-22% of such irradiated patients.

The inventors have surprisingly found that when EPO is bound locally to a membrane, it seems to act directly on the osteoblasts, and thereby improves their viability. This effect can be used in order to provide an efficient therapy and/or prevention of osteonecrosis, such as BP-ONJ, and BP-ONJ as a side-effect of an anti-cancer bisphosphonate therapy as described herein.

In yet another aspect of the present invention, said EPO is applied locally to said patient. Said EPO can thus be administered in the form of an ointment, a cream, a powder, an emulsion, a gel, a glycerogelatine, a paste, a plaster, a membrane, a sprayable composition or a lotion.

In the context of a bone reconstruction before or during the implantation, in particular for the so-called "guided bone regeneration" (GBR), in surgical peridontal therapies, often the use of membranes is mandatory. Using membranes first leads to a stabilization of the augmentation, and also the unwanted ingrowth of soft tissues is avoided by the membrane ("occlusion").

Absorbable membranes are in use in medicine for about 40 years. In addition to the experimental use of polylactide-membranes in the context of neurosurgery at the spine, polylactide-membranes were developed that can be degraded by the body into its biological main component lactate (Robert PM, Frank RM. Periodontal guided tissue regeneration with a new resorbable polylactic acid membrane. J Periodontol. 1994 May;65(5):414-22). Self-dissolving membranes had also been tested in the dental field (Lee CK, Alexander M. Prevention of postlaminectomy scar formation. Spine (Phila Pa 1976). 1984 Apr; 9(3):305-12). In 1988 and 1990, the use of absorbable membranes in the therapy of bone defects in periodontal disease was studied by Magnusson et al. (Magnusson I, Batich C, Collins BR. New attachment formation following controlled tissue regeneration using biodegradable membranes. J Periodontol. 1988 Jan; 59(1):1-6; Magnusson I, Stenberg W, Batich C, Egelberg J. Connective tissue repair in circumferential periodontal defects in dogs following use of a biodegradable membrane. J Clin. Periodontol. 1990 Apr; 17(4):243-8.). It was shown that the use of membranes led to a significant increase of the bone regeneration at the root of the tooth. Based on this, clinical trials were undertaken in humans to study the use of collagen membranes in bone regeneration (Chung KM, Salkin LM, Stein MD, Freedman AL. Clinical evaluation of a biodegradable collagen membrane in guided tissue regeneration. J Periodontol. 1990 Dec; 61(12):732-6).

Therefore, according to the invention, the EPO is preferably applied bound to a biodegradable membrane, such as, for example, bound to a collagen membrane.

Nevertheless, absorbable collagen membranes merely stabilize the defect, without providing an increased bone formation through the occlusion in order to promote guided bone regeneration. Furthermore, the use of these membranes often requires a surgical intraoperative adjustment of the membrane and a fixation using pins. This led to the development of membranes on the basis of a hydrogel, which is self-curing after the application thereof (Hu BH, Messersmith PB. Enzymatically cross-linked hydrogels and their adhesive strength to biosurfaces. Orthod Craniofac Res. 2005 Aug;8(3):145-9.). In a PEG-hydrogel application a simplification of the surgical process, in particular a better integration und fixation of the bone replacement material and the membrane can be achieved. Furthermore, there is no more need to remove the membrane, which excludes the high risk of an injury of the soft tissues.

In order to combine the advantage of the resorption with occlusion, a so-called polyethylene glycol-membrane (PEG membrane) was developed, which - following the application of the gel-like liquid - cures, and thus provides an effective barrier-function for fibroblasts. A removal of the membrane is not required, since it is degraded and absorbed by the body. In 2010, a self-curing PEG membrane (Straumann MembraGel^{™}) for guided dental bone regeneration (GBR) became available.

The polyethylene glycol building blocks of such a membrane form a tight, occlusive surface, thus, the membrane can be used as a carrier for therapeutics, and for an occlusion of leaks of the dura in the context of neurosurgical procedures, as well as for vessel sutures.

The PEG membrane has a markedly higher barrier function and occlusion than other known and collagen membranes. Upon an analysis of the degradation processes and degradation of the PEG-polymers through hydrolysis, no unwanted immune reactions could be found. Therefore, PEG membranes are biologically safe, although they have a slightly delayed degradation compared to conventional collagen membranes (Herten M, Jung RE, Ferrari D, Rothamel D, Golubovic V, Molenberg A, Hämmerle CH, Becker J, Schwarz F. Biodegradation of different synthetic hydrogels made of polyethylene glycol hydrogel RGD-peptide modifications: an immunohistochemical study in rats. Clin Oral Implants Res. 2009 Feb; 20(2): 116-25).

Preferred is the use of the EPO according to the present invention, bound to biodegradable membranes (e.g. collagen membranes (BioGuide, Geistlich) or self curing gel-membranes (e.g. Membragel^{™}, Straumann). In case of the solid membrane, either an immersion of the membrane with EPO is possible, as well as a biochemical attachment of the EPO to the membrane e.g. using covalent bonds. Additional biochemical methods of attaching the hydrophobic, temperature and pH-stabile glycoprotein are also feasible.

In yet another preferred aspect of the present invention, the EPO according to the present invention is thus applied in a self-curing gel membrane.

In case of self curing membranes, in addition to the fixation to the monomers a merely passive addition to the mix-system of, e.g. Membragel^{™}, is possible. The use of Membragel^{™} for "guided bone regeneration" in connection with implantological surgeries and the periodontal therapy has already been investigated and documented. The use of native Membragel^{™} in the context of bisphosphonate associated osteonecrosis has not been proposed.

Therefore, yet another preferred aspect of the present invention relates to the use of a pharmaceutical composition comprising EPO in hydrogel membranes for bone augmentation and regeneration, and the treatment of conditions associated with bone loss, preferably BP-ONJ.

In yet another preferred aspect of the present invention, the EPO according to the present invention is applied to the patient together with at least one additional bone-improving substance. "Together" shall mean either as a combination of the at least two active components (i.e. EPO and bone-improving substance), or as separate dosage forms that function together at the site of treatment. Examples are pharmaceutical substances, and/or biological preparations, like bone-forming cells, and other pharmaceutical substances e.g. ascorbic acid or mevalonate pathway metabolites like geranylgeraniol. l.

Yet another preferred aspect of the present invention then relates to the use of the EPO according to the present invention in guided bone regeneration as described herein.

Another aspect of the present invention then relates to a method for preventing and/or treating osteonecrosis in a patient, comprising administering to said patient an effective amount of an erythropoietin (EPO) according to the present invention as above.

Another aspect of the present invention then relates to a method for preventing and/or treating radioosteonecrosis in patient after radiotherapy.

Thereby, according to the invention, the EPO is preferably applied bound to a biodegradable membrane, such as, for example, bound to a collagen membrane, or is applied in a self-curing gel membrane, as described above.

Preferred is a method for preventing and/or treating osteonecrosis in a patient according to the present invention, wherein said EPO is applied together with at least one additional bone-improving substance as described above.

The present invention will now be described further in the following examples, nevertheless, without being limited thereto. For the purpose of the present invention, all references as cited are incorporated by reference in their entireties.
Figure 1 shows the effect of EPO on osteoblasts.

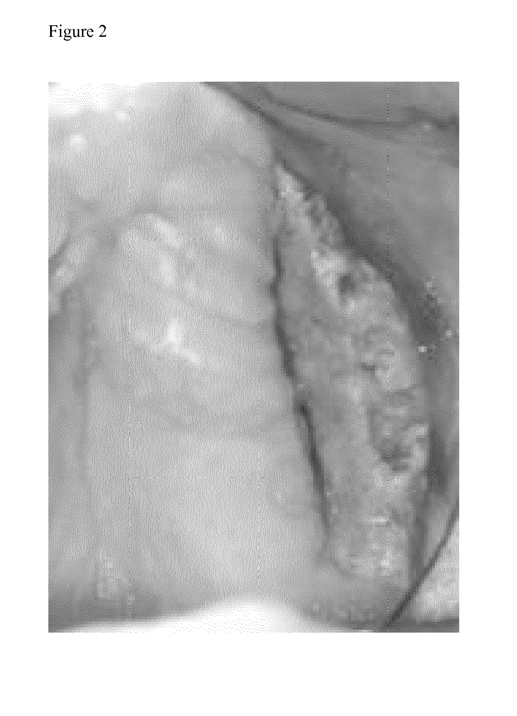

Figure 2 shows a photo of the clinical picture BP-ONJ of the upper jaw.

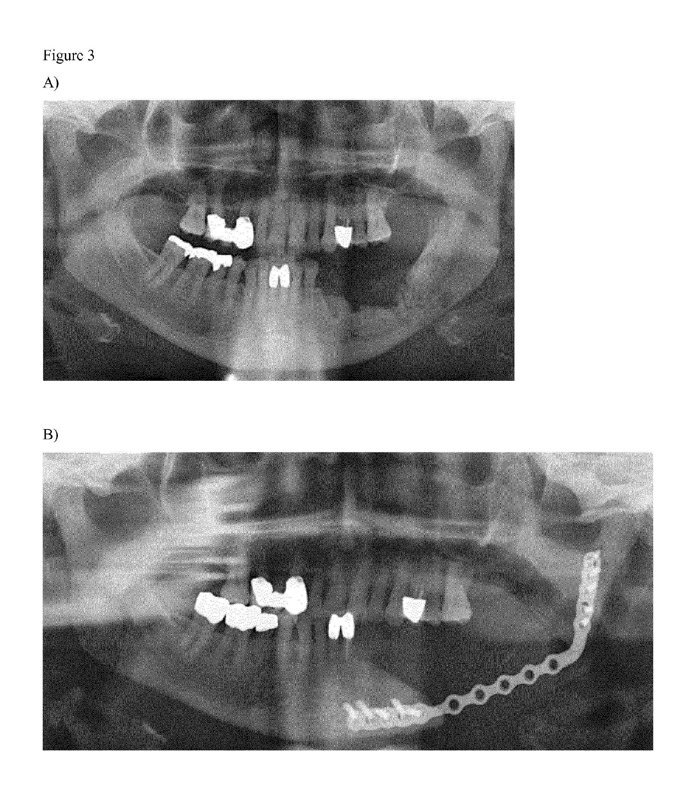

Figure 3 shows x-ray images of a partial resection of the jaw by BP-ONJ (A), and the subsequent surgical fixation (B)

### Examples

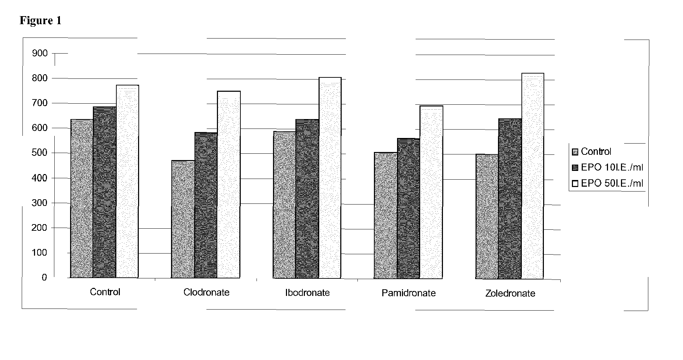

### Increase of viability of cells through the application of EPO (Figure 1)

It was shown in earlier experiments that bisphosphonates have a detrimental effect on the cells of the jaw. Since an in vivo model of the osteonecrosis of the jaw is missing, the inventors established and performed different cell culture experiments.

The experiments show that bisphosphonates interfere with the physiological cellular functions of cells of the solid and bone tissues (osteoblasts) as well as of the soft tissues (fibroblasts and blood vessel cells). These results reflect the clinical picture of an avascular osteonecrosis that can be found in bisphosphonate induced osteonecrosis of the jaw.

Cells were incubated with either non-nitrogen containing bisphosphonates, such as Clodronate, or nitrogen containing bisphosphonates, such as Zoledronate. The concentration of the bisphosphonates was at 50 µmol, and the drug was dissolved in PBS. Osteoblasts were used that were held at standard conditions. The control did not receive bisphosphonates. On order to measure the viability, the MTT test was used. The results are shown in Figure 1.

Upon the addition of erythropoietin (10 I.E or 50 I.E.), a dose-dependent increase of the viability of the cells was found. Furthermore, the specific inhibition of the cells (osteoblasts, fibroblasts, HUVEC, and keratinocytes) was significantly reduced in particular by the nitrogen containing bisphosphonates. Thus, a both healing of the osteonecrosis, such as the BPONJ, or the combined use following surgical interventions becomes possible.

The results furthermore show that EPO can also be used in order to prevent osteonecrosis, in particular in the context of a bisphosphonate therapy.

## Claims

1. Topically applied erythropoietin (EPO) for the prevention and/or treatment of osteonecrosis in a patient.

2. The EPO according to claim 1, wherein said patient is a cancer patient, and preferably a cancer patient undergoing an anti-cancer bisphosphonate therapy.

3. The EPO according to claim 1 or 2, wherein said osteonecrosis is selected from BP-ONJ, BP-ONJ as a side-effect of an anti-cancer bisphosphonate therapy, and radioosteonecrosis.

4. The EPO according to any of claims 1 to 3, wherein said EPO is applied locally to said patient.

5. The EPO according to any of claims 1 to 4, wherein said EPO is applied bound to a biodegradable membrane, such as, for example, bound to a collagen membrane.

6. The EPO according to any of claims 1 to 4, wherein said EPO is applied in a self-curing gel membrane.

7. The EPO according to any of claims 1 to 6, wherein said EPO is applied together with at least one additional bone-improving substance and/or biological preparations, like bone-forming cells, ascorbic acid or mevalonate pathway metabolites, like geranylgeraniol.

8. The EPO according to any of claims 1 to 7 for use in the guided bone regeneration.

9. A method for preventing and/or treating osteonecrosis in a patient, comprising administering to said patient an effective amount of an erythropoietin (EPO) according to any of claims 1 to 6.

10. The method according to any of claims 1 to 6, w wherein said EPO is applied together with at least one additional bone-improving substance.
